# EUROPÄISCHE PATENTANMELDUNG

(11) **EP 1 034 773 A2**
(43) Veröffentlichungstag der Anmeldung: **13.09.2000**
(21) Anmeldenummer: 00104901.4
(22) Anmeldetag: 08.03.2000
(51) Int. Cl.: A61K 6/04

(54) **Dental-Legierung**

(30) Priorität: 12.03.1999 DE 19911166
(71) Anmelder: BEGO Bremer Goldschlägerei Wilh. Herbst GmbH & Co., 28359 Bremen (DE)
(72) Erfinder: Strietzel, Roland, Dr., 28865 Lilienthal (DE)
(74) Vertreter: Eisenführ, Speiser & Partner

(57) **Zusammenfassung**

Eine Edelmetall- oder Nichtedelmetall-Legierung für Dentalzwecke enthält Zirkonium zwischen 0,05 bis 6 %.

## Beschreibung

Die Erfindung betrifft Edelmetall- oder Nichtedelmetall-Legierungen für Dentalzwecke.

Praktisch allen bekannten und üblichen Dental-Legierungen ist gemeinsam, daß sie neben den Basiselementen - einerseits Gold, Palladium, Silber, andererseits Kobalt, Nickel - für die (jeweils) geforderten Eigenschaften Legierungselemente aufweisen, welche diese Eigenschaften herbeiführen sollen. So werden zur Verbesserung der mechanischen Eigenschaften den Edelmetall-Legierungen - je nach Basismetall - Platin, Palladium, Silber, Kupfer, Zinn, Zink, Indium, Gallium, Mangan und Eisen, den Nichtedelmetall-Legierungen Niob, Tantal, Wolfram und/oder Titan beigegeben. Als Kornfeiner dienen bei Edelmetall-Legierungen die Metalle der Platingruppe sowie Rhenium, Tantal und Titan, bei Nichtedelmetall-Legierungen ebenfalls Titan und Tantal sowie Wolfram.

Weil die aus Dental-Legierungen hergestellten Gußstücke (Onlays, Kronen, Brücken etc.) häufig mit einer keramischen Verblendung versehen werden sollen, die namentlich durch Sauerstoffbrücken (Oxide) ihre Verbindung zum Metallgerüst erhalten, werden der Dental-Legierung meist Haftoxidbildner zugemischt, im Falle von Edelmetall-Legierungen Indium, Gallium, Zinn und Eisen, im Falle von Nichtedelmetall-Legierungen Chrom, Gallium, Eisen und Lanthanoide. Während bei Edelmetall-Legierungen Maßnahmen zur Korrosionsresistenz entfallen, enthalten Nichtedelmetall-Legierungen aus diesem Grunde Chrom, Molybdän sowie Wolfram und/oder Niob; mehr oder weniger spekulativ ist auch die Beigabe von Zirkonium vorgeschlagen worden.

Es wurde nun gefunden, daß ein Zusatz von - je nach Basislegierung und Einsatzzweck - 0,05 bis 6 (Massen-)% an Zirkonium überraschend signifikante Verbesserungen wichtiger physikalischer Eigenschaften von Dental-Legierungen bewirkt, und zwar in komplexer Weise derart, daß mit einer Erhöhung der Warmfestigkeit und der Korrosionsresistenz eine Optimierbarkeit der mechanischen Festigkeit einhergeht, also nicht - wie meist zu beobachten - ein Vorteil in einem Punkt mit einem Nachteil in einem anderen Punkt erkauft werden muß. So bedarf es aufgrund der erhöhten Warmfestigkeit keines - häufig abgelehnten - Palladiumanteils mehr, um die Stabilität des Metallgerüsts beim Aufbringen der Keramik mit einer Temperatur von 900 - 1000 °C zu gewährleisten, und es kann wegen der erhöhten Korrosionsresistenz im Falle von Nichtedelmetall-Legierungen auf die Zugabe korrosionsanfälliger Metalle verzichtet werden.

Vor allem aber wirkt sich der Ersatz von Titan durch Zirkonium vorteilhaft aus. Beide Metalle sind zwar chemisch verwandt und haben vergleichbare Eigenschaften, jedoch sind - aufgrund des größeren Atomradius' von Zirkonium gegenüber Titan - die Einflüsse durch Mischkristallbildung beim Zirkonium weniger ausgeprägt. Der deshalb benötigte größere Anteil verringert den Einfluß des Zirkonium-Verzunderns beim Wiedervergießen und erlaubt eine Steuerung der Einflüsse. Dental-Legierungen mit Zirkoniumgehalt sind deshalb auf ihren Einsatzzweck "einstellbar": Für ein Inlay benötigt man beispielsweise nicht die hohen Festigkeitswerte wie für eine Brücke, bei der eine geringe Festigkeit die Haltbarkeit einer aufgebrachten Keramikverblendung gefährden würde.

Es kommt hinzu, daß Zirkonium nicht nur als Kornfeiner einen vorteilhaften Einfluß auf die Legierung ausübt, sondern mit seinem Oxid auch einen guten Haftoxidbildner darstellt, so daß eine Legierung mit Zirkonium besonders für die Herstellung von Gerüsten geeignet ist, welche mit modernen Zirkoniumdioxid-haltigen Keramikmassen verblendet werden sollen. Ferner verhindert die von einem Zirkoniumgehalt der Dental-Legierung gebildete Oxidschicht eine Verfärbung von Keramikverblendungen, wenn das Gerüst aus einer Silberlegierung besteht, weil jene Oxidschicht eine Diffusion des Silbers in die Keramik verhindert. Insoweit kann das Zirkonium die Zugabe von Indium ersetzen, wobei ein geringerer Gehalt von Zirkonium ausreicht als im Falle der Indium-Beigabe erforderlich wäre.

Schließlich zeichnet sich ein aus einer der erfindungsgemäßen Legierungen hergestellter Dental-Gußkörper durch eine hohe Ästhetik aus, wenn er poliert wird.

### Beispiele:

1. Eine hochgoldhaltige Legierung für Inlays oder kleine Onlays besteht aus 95,9 % Gold, 4,0 % Zirkonium und 0,1 % Tantal.
2. Eine ebenfalls hochgoldhaltige Dental-Legierung für Primär-Kronen für teleskopierende Arbeiten besteht aus 99 % Gold, 0,5 % Zirkonium und 0,5 % Zink.
3. Eine Silber-Gold-Dental-Legierung für Inlays, Onlays und kleine Vollgußbrücken besteht aus 58,9 % Silber, 35,5 % Gold, 4,0 % Zinn, 1,0 % Zink und 0,5 % Zirkonium.
4. Eine Silber-Dental-Legierung für Kronen sowie Brücken für die keramische Verblendung besteht aus 58,9 % Silber, 35,0 % Palladium, 4,0 % Zinn, 1,0 % Zink, 0,5 % Gold und 0,5 % Zirkonium.
5. Eine aufbrennfähige Kobalt-Chrom-Legierung für Kronen und Brücken besteht aus 68,5 % Kobalt, 24,0 % Chrom, 4,5 % Molybdän und 3 % Zirkonium.
6. Eine aufbrennfähige Nickel-Chrom-Legierung für Kronen und Brücken besteht aus 61,4 % Nickel, 22,9 % Chrom, 8,8 % Molybdän, 3,9 % Niob, 2,1 % Eisen, 0,5 % Zirkonium und 0,4 % Mangan.

## Patentansprüche

1. Edelmetall- oder Nichtedelmetall-Legierung für Dentalzwecke, gekennzeichnet durch einen Gehalt an Zirkonium von 0,05 %, insbesondere 0,25 bis 6 %.

2. Edelmetall-Legierung nach Anspruch 1, dadurch gekennzeichnet, daß sie 0,1 % bis 1,0 % Zirkonium enthält.

3. Edelmetall-Legierung nach Anspruch 1, dadurch gekennzeichnet, daß sie 3,0 % bis 5,0 %, insbesondere 4,0 % Zirkonium enthält.
